## Europäisches Patentamt

## European Patent Office

## Office européen des brevets

(11) Veröffentlichungsnummer: **0 084 805**
**B1**

(12) **EUROPÄISCHE PATENTSCHRIFT**

(45) Veröffentlichungstag der Patentschrift:
**16.10.85**

(21) Anmeldenummer: **83100211.8**

(22) Anmeldetag: **12.01.83**

(51) Int. Cl.⁴: **C 09 J 3/00,** C 08 L 33/06,
C 08 F 4/44, A 61 K 6/08

(54) **Aerob härtende Kunststoffmassen.**

(30) Priorität: **21.01.82 DE 3201731**

(43) Veröffentlichungstag der Anmeldung:
**03.08.83 Patentblatt 83/31**

(45) Bekanntmachung des Hinweises auf die Patenterteilung:
**16.10.85 Patentblatt 85/42**

(84) Benannte Vertragsstaaten:
**AT BE CH DE FR GB IT LI NL SE**

(56) Entgegenhaltungen:
**EP - A - 0 051 796**
**US - A - 4 167 616**

(73) Patentinhaber: **Henkel Kommanditgesellschaft auf
Aktien, Postfach 1100 Henkelstrasse 67,
D-4000 Düsseldorf-Holthausen (DE)**

(72) Erfinder: **Ritter, Wolfgang, Dr., Hochdahler Strasse 32,
D-4010 Hilden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beschreibung

Die Erfindung bezieht sich auf aerob härtende Kunststoffmassen, die beispielsweise als Giessharze oder Füllstoffe, insbesondere aber als Reaktionsklebstoffe Verwendung finden können. Im allgemeinen liegen diese Massen als lagerstabile Mehrkomponentensysteme vor, die zur formgebenden Verarbeitung miteinander vermischt oder in anderer Weise vereinigt werden und dann bei Luftzutritt härten. Beispielhaft sei auf Reaktionsklebstoffe verwiesen, die eine erste Komponente mit ethylenisch ungesättigten Monomeren neben einer zweiten Komponente – dem Härter – enthalten, wobei nach Vereinigung dieser Komponenten an der Luft die Härtung durch radikalische Polymerisation eintritt. Die Erfindung wird im folgenden anhand solcher mehrkomponentiger Klebstoffsysteme geschildert, gilt jedoch sinngemäss auch für andere aerob härtende Kunststoffmassen.

Klebstoffe, die durch Polymerisation von ethylenische Gruppen enthaltenden Verbindungen härten, sind seit langem bekannt. Diese lassen sich beispielsweise aus Methacrylsäureestern bzw. aus anderen Derivaten von Acrylsäure oder α-substituierten Acrylsäuren durch Zusatz von Peroxiden bzw. Hydroperoxiden und weiteren Hilfsmitteln herstellen. Die Klebstoffkomponente besteht üblicherweise aus der Lösung eines Elastomeren in einem Monomeren. Soll die Härtung bei Raumtemperatur oder nur mässig erhöhten Temperaturen stattfinden, so müssen Beschleuniger für den Peroxidzerfall eingesetzt werden, üblicherweise aromatische Amine oder eine Schiffsche Base.

Für spezielle Anwendungsgebiete, insbesondere für dentalmedizinische oder chirurgische Anwendungen, sind Binde- und Füllmittel bekannt, die neben (Meth)-acrylsäureestern und weiteren ethylenische Doppelbindungen enthaltenden Reaktionspartnern als Härterkomponente Trialkylborverbindungen einsetzen. Derartige Trialkylborverbindungen lösen die Polymerisation bei Luftzutritt unter Normaltemperatur aus, sie weisen aber den Nachteil auf, leicht entzündlich zu sein, so dass die Handhabung dieser Reaktionmassen bzw. Klebstoffe erhebliche Schwierigkeiten bereitet. Man hat versucht, diesen Nachteil dadurch zu beseitigen, dass man die Trialkylborverbindungen mit 0,3 bis 0,9 Mol Sauerstoff umgesetzt hat. Auch ist versucht worden, die Trialkylborverbindungen mit Aminen umzusetzen, um so die Selbstentzündlichkeit herabzusetzen. Durch diese Massnahmen wird die Zündtemperatur zwar in einen Bereich von 0 bis 70°C verschoben, gleichwohl bleibt eine erhebliche Unsicherheit bei der Handhabung derartiger Mischungen. Ausserdem ist die Reaktivität dieser Derivate stark vermindert.

Freie Boralkylverbindungen werden bei Zutritt von überschüssigem Sauerstoff zu Borsäureestern oxidiert, die nicht mehr polymerisationsauslösend wirken. Die Anwendung und insbesondere auch die Dosierung bisher bekannter Boralkyle als Initiatoren erfordert – ebenso wie selbstverständlich auch der Herstellungsprozess – völligen Sauerstoffausschluss. Konkret muss die jeweils benötigte Substanzmenge unter Inertgas in völlig dichte Gefässe abgepackt und Sauerstoffzutritt zum Vorratsgefäss ausgeschlossen werden. Die portionierten Boralkyle sind quantitativ zu verbrauchen. Die bisher beschriebenen Systeme eignen sich damit nicht für allgemeine konstruktive Verklebungen beispielsweise beim Verbinden von Metall, Holz, Glas, Keramik und/oder Kunststoffen. Aber auch ihr Einsatz auf den genannten Spezialgebieten unterliegt beträchtlichen Einschränkungen.

Es hat daher bereits Bemühungen gegeben, Borverbindungen in Form von Oligomeren oder Polymeren einzusetzen. So wird in dem US Patent 4167616 vorgeschlagen, ausser niedermolekularen Diboranaddukten Oligomere zu verwenden, deren wiederkehrende Einheiten zumindest zwei Boratome aufweisen, die über Kohlenwasserstoffgruppen verbunden sind und bei denen aliphatische C-Atome zumindest an ein Boratom gebunden sind. Derartige Oligomere tragen in der Hauptkette B–C-Bindungen. Sie zerfallen daher bei der Polymerisationsauslösung, wobei die schützende Funktion des polymeren Gerüstes verlorengeht und darüber hinaus niedermolekulare Bruchstücke entstehen, die bei manchen Anwendungen nicht erwünscht sind. Dies gilt auch für höhermolekulare Produkte des gleichen Aufbaus, wobei hier als weiterer Nachteil die geringe Löslichkeit auf Grund von Vernetzungen mit hinzukommen kann.

Die vorliegende Erfindung geht von der Aufgabe aus, aerob härtende Kunststoffmassen zu schaffen, die insbesondere auch schon bei Raumtemperatur und Luftzutritt härten, sich dabei gefahrlos handhaben lassen und bei praktikablen Topfzeiten zu guten Materialeigenschaften, beispielsweise zu guten Verklebungen, führen. Die Erfindung will dabei insbesondere zweikomponentige Systeme schaffen, die beispielsweise auf dem Gebiet der Füllstoffe, der Giessharze und insbesondere aber der Klebstoffe neuartige Arbeitsmöglichkeiten eröffnen. Im engeren Sinne geht die Erfindung von der Aufgabe aus, neuartige Härter auf Basis von Organoborverbindungen einzusetzen, die mit ethylenisch ungesättigten polymerisierbaren Reaktionskomponenten unter den angegebenen Bedingungen reagieren.

Die Erfindung betrifft damit beispielweise zweikomponentige Reaktionsklebstoffe, die nach Vermischung von Härter und polymerisierbarer Komponente appliziert werden können. In den Rahmen der Erfindung fallen aber auch die sogenannten «No-Mix-Klebstoffe» oder «Acrylat-Klebstoffe der zweiten bzw. dritten Generation» (vgl. hierzu «Adhesives Age» 1976 Nr. 9, 21 bis 24 sowie «Adhesion», Nr. 3 1981, 156 bis 161). Hier kann auf die getrennte Vermischung der Klebstoffkomponente und der Härterkomponente verzichtet werden. Zur Anwendung wird vielmehr die Initiatorkomponente in dünner Schicht auf eine oder beide der zu verklebenden Oberflächen aufgebracht. Nach einer Wartezeit, die bis zu Stunden betragen kann, wird die Klebstoffkomponente aufgetragen. Die Verklebung erfolgt durch Fixierung der Teile in der gewünschten Position. Die Erfindung geht weiterhin von der Aufgabe aus, Klebstoffe bzw. Füllstoffe zu finden, die sich bei gefahrloser Handhabung als Klebmittel im dentalmedizinischen Bereich sowie als Binde- und Klebmittel in der Chirurgie einsetzen lassen. Insbesondere sollen diese Reaktionsklebstoffe sich auch auf Metallflächen verwenden lassen und für die Klebung von Knochen bzw. Zähnen oder sonstigem harten lebenden Gewebe geeignet sein.

Die Erfindung will insbesondere eine neue Härterkomponente auf Basis von Organoborverbindungen zur Verfügung stellen, die bei der Zugabe zu polymerisierbaren Monomeren durch Oxydation mit Luftsauerstoff eine schnelle und leicht steuerbare Polymerisation auslösen. Im Gegensatz zu den bisher bekannten Systemen sollen die neuen Organoborverbindungen als solche kaum oder nicht sauerstoffempfindlich, in gar keinem Fall aber selbstentzündlich sein. Die Erfindung will insbesondere auch solche Härter auf Basis von Organoborverbindungen zur Verfügung stellen, die bei der Lagerung an Luft unter Normaltemperaturen stabil bleiben und dennoch bei Zugabe zu polymerisierbaren Monomersystemen spontan die Polymerisation auslösen.

Gegenstand der Erfindung sind dementsprechend in einer ersten Ausführungsform aerobhärtende Kunststoffmassen, wie Giessharze, Füllstoffe und insbesondere Reaktionsklebstoffe auf Basis polymerisierbare ethylenische Doppelbindungen enthaltender Systeme und Organo-Borverbindungen als Polymerisationsinitiatoren, dadurch gekennzeichnet, dass sie polymere Organo-Borverbindungen enthalten, die als Substituenten an einer gegen Luftzutritt stabilen Polymermatrix Organo-Borreste aufweisen, wobei als Polymermatrix ein ethylenische Doppelbindungen aufweisendes Oligomeres bzw. Polymeres vorliegt, dessen Doppelbindungen wenigstens anteilsweise durch Einführung der borhaltigen Substituenten mittels Hydroborierung zu gesättigten Bindungen umgewandelt sind.

Die borhaltigen Reste sind über $B-C$-Bindungen an die Polymermatrix gebunden. Diese borhaltigen Substituenten der Polymermatrix sind in einer weiterhin bevorzugten Ausführungsform ihrerseits am Bor mit wenigstens einer weiteren $B-C$-Bindung an einen oder mehrere organische Reste gebunden. Bevorzugte Reste sind hier Kohlenwasserstoffreste, die allerdings auch Heteroatome insbesondere O, N und/oder S enthalten können. Geeignete Substituenten am Bor sind insbesondere Alkyl-, Cycloalkyl- und/oder Arylreste, die in einer oder in den beiden nicht durch die Polymermatrix besetzten Valenzen des Bors vorliegen können. Liegen solche von Wasserstoff abweichenden organischen Reste in beiden Borvalenzen vor, so können sie ihrerseits zu einem Ringsystem geschlossen sein.

Der Kern der Erfindung liegt damit in der Verwendung einer neuen Klasse oligomerer bzw. polymerer Organoborverbindungen als Polymerisationsinitiatoren bzw. als Härter für die ethylenische Doppelbindungen enthaltenden polymerisierbaren Systeme. Gegenüber herkömmlichen Boralkylhärtern besitzen die erfindungsgemäss eingesetzten oligomeren bzw. polymeren Borverbindungen wesentliche Vorteile. Sie sind nicht selbstentzündlich und stellen geringe Anforderungen bei der Lagerung. Die Aktivität dieser Härter bleibt selbst bei längerer Lagerung an der Luft erhalten. Die Verträglichkeit der polymerisierbaren Komponente mit dem Härter kann durch geeignete Ausgestaltung der Polymermatrix für jeden Fall leicht sicher gestellt werden. Im allgemeinen ist die zur Härtung der Monomerkomponente benötigte Menge der oligomeren bzw. polymeren Organoborverbindung äusserst niedrig.

Die erfindungsgemäss als Polymerisationsinitiatoren eingesetzten Borverbindungen können in einfacher Weise erhalten werden, indem Oligomere bzw. Polymere, die additionsbereite Kohlenstoff-Doppelbindungen enthalten, der Hydroborierung unterworfen und hierdurch die borhaltigen Reste an wenigstens einem Teil der additionsbereiten Doppelbindungen eingeführt werden. Zur Hydroborierung geeignet sind sowohl mono- als auch disubstituierte Borane, d.h. Verbindungen der allgemeinen Formel $R_1R_2BH$, wobei in dieser Formel $R_1$ ein organischer Rest, bevorzugt ein Kohlenwasserstoffrest, und $R_2$ Wasserstoff oder ebenfalls ein organischer Rest ist, der mit $R_1$ gleich oder von ihm verschieden sein kann oder auch mit $R_1$ und dem Bor gemeinsam ein Ringsystem bildet.

Polymere Organoborverbindungen der erfindungsgemäss eingesetzten Art sind als solche bisher nicht beschrieben. In der Literatur finden sich vereinzelte Hinweise auf ihre intermediäre Bildung bei der Synthese bestimmter polymerer Verbindungen. Verwiesen wird beispielsweise auf «Makromol. Chem.» 178, 2837 bis 2842 (1977). Beschrieben werden hier die Synthese und Strukturuntersuchung des Poly(1-hydroxytetramethylen)s durch Hydroborierung des 1,4-Polybutadiens mit 9-Borabicyclo[3.3.1]-nonan (9-BBN) mit unmittelbar anschliessender Oxidation und Hydrolyse der intermediär gebildeten polymeren Organoborverbindung zum hydroxylierten Kohlenwasserstoffpolymeren. Eine Isolierung der im lösungsmittelhaltigen Reaktionsgemisch intermediär entstehenden polymeren Organoborverbindung hat nicht stattgefunden. Entsprechend werden keine Angaben über die Eigenschaften des borhaltigen Polymeren gemacht. Die Erfindung geht von der überraschenden Erkenntnis aus, dass die Fixierung der geschilderten borhaltigen Reste an einer an sich gegen Zutritt von Luftsauerstoff stabilen oligomeren oder polymeren Matrix zu einem neuen Typ von Organoborverbindungen führt, der sich in technisch wesentlichen Eigenschaften von den bisher beschriebenen und in Sonderfällen auch genutzten Boralkylverbindungen unterscheidet. Besonders auffallend ist die relative Stabilität der erfindungsgemäss beschriebenen polymeren bzw. oligomeren Organoborverbindungen gegen Zutritt von Luftsauerstoff, wodurch ihre praktische Handhabung und Verwendung als Polymerisationsinitiatoren wesentlich erleichtert wird.

Die der Hydroborierung zugängliche ethylenische Doppelbindungen aufweisende Polymermatrix kann je nach Struktur und Molekulargewicht niedrigviskos fliessfähig bis fest sein. Ihr mittleres Molekulargewicht kann bis zu Werten von einigen Millionen reichen und liegt üblicherweise im Bereich von etwa 150 bis 3 Millionen. Niedrigere Werte innerhalb dieses Bereiches sind häufig bevorzugt, beispielsweise solche im Bereich von etwa 300 bis 500 000 und insbesondere solche im Bereich von etwa 500 bis 10 000. Für einige Anwendungszwecke — beispielsweise auf dem Gebiet der Reaktionsklebstoffe — kann es wünschenswert sein, dass die Polymermatrix und auch die daraus gewonnenen polymeren Organoborverbindungen bei Raumtemperatur viskos fliessfähig bzw. streichfähig sind. Hier können beispielsweise Molekulargewichte der Polymermatrix im Bereich von etwa 300 bis 3000 besonders geeignet sein. Für die Wirksamkeit der erfindungsgemäss eingesetzten polymeren Organoborverbindungen als Initiatoren ist das allerdings keine Voraussetzung. Im Gegenteil kann die Lagerstabilität von bei Raumtemperatur festen entsprechenden polymeren Organoborverbindungen besonders gut sein.

Die polymere Matrix kann vor der Hydroborierung in beliebig starkem Ausmass ethylenisch ungesättigt sein. Bevorzugt sind entsprechende Materialien, die vor der Hydroborierung eine Jodzahl im Bereich von etwa 1 bis 500 aufweisen. Innerhalb dieses Bereiches sind besonders bevorzugte Werte der Jodzahl von etwa 5 bis 100 und insbesondere von etwa 8 bis 50.

Die der Hydroborierung zugänglichen ethylenischen Doppelbindungen können im Ausgangspolymeren in der Hauptkette und/oder in Seitenketten-Substituenten vorliegen. Bei einzelnen im Folgenden noch zu schildernden polymeren Typen bieten sich insbesondere in Seitenketten-Substituenten vorliegende Doppelbindungen für die Hydroborierung zum Polymerisationsinitiator an.

Die Polymermatrix kann vor der Hydroborierung geradkettige oder verzweigte Struktur aufweisen, aber auch polymere Materialien mit vernetzter Struktur sind nicht ausgeschlossen. Während die zuerst genannten Polymertypen zur Einführung der borhaltigen Gruppen üblicherweise in Lösungsmitteln umgesetzt werden, können unlösliche vernetzte Polymere, die noch reaktive Doppelbindungen aufweisen, in feinstverteiltem, vorzugsweise in Lösungsmitteln gequollenem Zustand der Umwandlung zur borhaltigen Matrix unterworfen werden. Hier kann beispielsweise die Umsetzung in Suspension bzw. Dispersion des feinstteiligen vernetzten Matrixmaterials in einem inerten Lösungsmittel vorgenommen werden.

Als Polymermatrix eignen sich grundsätzlich alle Polymertypen, soweit sie der Hydroborierung zugängliche Doppelbindungen aufweisen und keine reaktiven Gruppen besitzen, die bei der Einführung der borhaltigen Gruppen in das Polymermaterial zu unerwünschten Nebenreaktionen führen.

Im Rahmen der Erfindung ist es dabei durchaus möglich, dass bei der Einführung der borhaltigen Reste in die Polymermatrix nicht nur ethylenische Doppelbindungen hydroboriert werden, sondern dass ein Anteil des borhaltigen Reagenzes auch mit anderen funktionellen Gruppen der Polymermatrix abreagiert. Als solche Gruppen sind beispielsweise Ketogruppen, Amidgruppen, Epoxidgruppen, und gegebenenfalls auch Estergruppierungen zu nennen. Wesentlich ist für die Einhaltung der erfindungsgemässen Lehre lediglich, dass auch ein hinreichender Anteil der ethylenischen Doppelbindungen der Hydroborierungsreaktion unterliegt, so dass die bororganischen Reste mit aerober Initiatorwirkung in ausreichendem Masse in der Polymermatrix ausgebildet werden.

Das Polymermaterial kann durch Polymerisation bzw. Copolymerisation olefinisch ungesättigter Komponenten, durch Polykondensation oder durch Polyaddition hergestellt worden sein. Durch an sich bekannte geeignete Auswahl der die Polymeren aufbauenden Monomertypen wird der erwünschte Gehalt an reaktiven Doppelbindungen für die anschliessende Hydroborierung im Polymermaterial sichergestellt. Besonders geeignet können als Polymermatrix ungesättigte Oligomere bzw. Polymere sein, die durch Polykondensation erhalten worden sind. In Betracht kommen hier alle bekannten Polykondensattypen wie Polyester, Polyamide, Polyimide, Polycarbonate, Polyurethane und dergleichen. Geeignet sind aber auch Oligomere bzw. Polymertypen, die durch Polyaddition erhalten werden.

Bevorzugt werden die Oligomeren bzw. Polymeren wie folgt hergestellt:

a) Durch Polymerisation von einem oder mehreren Dienen beziehungsweise Copolymerisation solcher Diene mit α-Olefinen.

b) Durch Polymerisation von Diolefinen, die im Molekül unterschiedliche reaktive olefinische Gruppen enthalten, beziehungsweise durch Copolymerisation solcher Di-Olefine mit α-Olefinen.

c) Durch Polymerisation von Vernetzern (mehrfach olefinisch ungesättigte Monomere) beziehungsweise durch Copolymerisation solcher Vernetzer mit α-Olefinen.

d) Durch Polyaddition von olefinische Gruppen enthaltenden cyclischen Ethern oder Iminen.

e) Durch Polykondensation von Olefingruppen enthaltenden Dicarbonsäuren mit Diolen oder Diaminen.

f) Durch Polykondensation von Dicarbonsäuren mit Olefingruppen enthaltenden Diolen oder Diaminen.

Die Polymerisation, Polyaddition beziehungsweise Polykondensation kann mit oder ohne Regelung des Molekulargewichts durchgeführt werden. Die entstehenden Produkte sind je nach der gewählten Monomerkombination und/oder Reglerkondensation niedrigviskos bis fest. Im einzelnen gilt hier das allgemeine Fachwissen der Polymerchemie. Im folgenden wird — ohne Beschränkung der Erfindung auf die speziell genannten Komponenten — eine Aufzählung von monomeren Reaktanten gegeben, die bei der Herstellung der olefinische Gruppen enthaltenden Oligomeren beziehungsweise Polymeren eingesetzt beziehungsweise mitverwendet werden, können.

*α-Olefine:*

Unsubstituierte geradkettige und/oder verzweigte α-Olefine mit 2 bis 25 C-Atomen, insbesondere mit 2 bis 10 C-Atomen, Vinylderivate wie Vinylester – zum Beispiel Vinylacetat, Vinylstearat, Vinylbenzoat, aber auch substituierte Verbindungen wie Vinyl-2-ethylhexoat, Vinyldichloracetat, Vinylcyanacetat, Vinyl-β-butoxypropionat, α-Methyl-vinylacetat und dergleichen, Vinylether – zum Beispiel Vinylmethylether, Vinylisobutylether, Vinyl-n-butylether, Vinylcyclohexylether, N-Vinyl-substituierte Verbindungen – beispielsweise Vinylpyrrol, Vinylcarbazol, Vinylindol, Vinylimidazol, Vinyldiphenylamin, Vinyl-phenyl-α-naphthylamin und dergleichen, N-Vinylsäureamide, -imide beziehungsweise N-Vinyl-lactame – zum Beispiel Vinylpyrrollactam, Vinyl-3-methylpyrrolidon, Vinyl-N-acetylanilin, Vinylsuccinimid, Vinyl-α-imid, Vinylmethylacetamid, Vinylpyrridin-Verbindungen – zum Beispiel 2-, 3- beziehungsweise 4-Methyl-Vinylpyridin, 5-Ethyl-2-Vinylpyridin und dergleichen, S-Vinylverbindungen, insbesondere Vinyl-substituierte Sulfide, Vinylthiolester, Vinylsulfoxide und Vinylsulfene, Vinylhalogenide, zum Beispiel Vinylchlorid, Acrylverbindungen wie Acrolein, Acrylsäure, Acrylsäurederivate insbesondere Ester beziehungsweise Amide der Acrylsäure und Acrylnitril.

*Diene:*

Geeignete Monomere sind beispielsweise 1,3-Butadien, Isopren, Cyclopentadien, Chloropren, 1,3-Pentadien, 2,3-Dimethylbutadien, 1,3-Hexadien oder 2,4-Hexadien.

*Diolefine:*

Diallylverbindungen beispielsweise Diallylsulfid, Diallylphthalat oder Diallylisocyanurat, ungesättigte Ester aus ungesättigten Monocarbonsäuren und Diolen beziehungsweise ungesättigte Amide aus ungesättigten Carbonsäuren und Diaminen. Beispiele ungesättigter Carbonsäuren sind etwa Acrylsäure, Methacrylsäure, Crotonsäure oder Undecylensäure.

*Olefinische Gruppen tragende cyclische Ether:*

Geeignet sind beispielsweise Vinyl-substituierte Epoxide beziehungsweise entsprechend substituierte cyclische Ether mit mehr als 2 benachbarten Kohlenstoffatomen im Ring Glycidylester ungesättigter Säuren wie Tetrahydrophthalsäure, Diglycidylester oder Verbindungen wie Vinylcyclohexenepoxid. Durch kationisch initiierte Reaktion – beispielsweise mittels Borfluorid oder seinen Komplexverbindungen – wird unter Öffnung des Ringethers die Polymerisation beziehungsweise Polyaddition ausgelöst. Die entstehenden oligomeren beziehungsweise polymeren Verbindungen enthalten die olefinischen Verbindungen zur nachfolgenden Reaktion mit Boranen. In an sich bekannter Weise kann durch Copolymerisation mit cyclischen Ethern beziehungsweise cyclischen Iminen, die keine funktionell reaktiven Olefingruppen im Molekül aufweisen, die Jodzahl im Oligomeren beziehungsweise Polymeren gesteuert werden.

*Ungesättigte Dicarbonsäuren:*

Dicarbonsäure, Maleinsäure, Fumarsäure, Mesaconsäure, Citraconsäure, Sorbinsäure, Alkenylbernsteinsäuren, zum Beispiel n-Octadecenyl-8-bernsteinsäure und Alkenylbernsteinsäureanhydride, Alkarylbernsteinsäureanhydride zum Beispiel n-Octadecenyl-8-bernsteinsäureanhydrid.

*Ungesättigte Diole:*

2,5-Dimethyl-3-hexen-2,5-diol, 2-Buten-1,4-diol sowie Diole, die eine olefinisch ungesättigte Funktion in einem Seitenkettensubstituenten enthalten. Analoges gilt für ungesättigte Diamine.

Als gesättigte Dicarbonsäuren, Diole beziehungsweise Diamine können alle bekannten Verbindungen der genannten Art eingesetzt werden, beispielsweise Oxalsäure, Malonsäure, Bernsteinsäure, Azelainsäure, Sebazinsäure, Phthalsäure, Hexahydrophthalsäure, Terephthalsäure, 2,3-Pyridin-Dicarbonsäure, 2,3-Chinolin-Dicarbonsäure, Diphenyldicarbonsäure und dergleichen. Beispiele für gesättigte Diole sind Ethylenglykol, Diethylenglykol, Triethylenglykol, Tetraethylenglykol, Propylenglykol, Dipropylenglykol, 1,3-Butylenglykol, 1,4-Butylenglykol, 1,4-Cyclohexandimethanol, 2-Methyl-1,4-butandiol, 1,6-Hexandiol, 1,10-Decandiol und dergleichen. Geeignete Diamine sind beispielsweise Ethylendiamin, Diethylentriamin, Triethylentetramin, Propylendiamin-1,2, Propylendiamin-1,3, Hexamethylendiamin, 1,5-Diaminopentan, 1,8-Diaminooctan, Diaminotoluol, 4,4,-Diamino-diphenylmethan und vergleichbare Diamine.

Für alle im Rahmen der Erfindung zum Einsatz kommenden Oligomeren bzw. Polymeren gilt, dass sie gegenüber dem Zutritt von Luftsauerstoff wenigstens unter Normaltemperatur beständig sind. Sie sind häufig überwiegend durch Ketten bzw. Kettenanteile mit C−C-Bindungen aufgebaut, wenngleich das auch keineswegs Vorbedingung ist. So sind beispielsweise Polyformale bekannte stabile Polymertypen mit alternierenden −C−O-Bindungen, die durch geeignete Modifizierung z.B. mit Seitenkettensubstituenten versehen werden können, in denen ethylenische Doppelbindungen zur nachfolgenden Hydroborierung vorliegen.

Das Ausmass der Hydroborierung an der Polymermatrix kann im Rahmen der insgesamt vorliegenden Doppelbindungen frei gewählt werden. Es hat sich allerdings als vorteilhaft erwiesen, wenn wenigstens ein substantieller Anteil dieser Doppelbindungen durch Einführung der borhaltigen Substituenten umgesetzt wird. So sind in bevorzugten Ausführungsformen der Erfindung wenigstens 30% und vorzugsweise wenigstens 50% der in der Polymermatrix ursprünglich vorhandenen ethylenischen Doppelbindungen hydroboriert. Besonders geeignet sind solche polymeren Organoborverbindungen, in denen wenigstens 80% vorzugsweise wenigstens 90% oder sogar wenigstens 95% der ethylenischen Doppelbindungen der Umsetzung mit den borhaltigen Komponenten zugeführt worden sind. Ein praktisch vollständig hydroboriertes Material ist in vielen Fällen der bevorzugte Initiator im Sinne der erfindungsgemässen Lehre.

Zur Hydroborierung kommen neben Diboran ($BH_3$) Organoboranverbindungen in Betracht, die einen oder zwei organische Reste insbesondere Kohlenwasserstoffreste aufweisen. Bevorzugte organische Reste sind hier Alkyl-, Cycloalkyl- und/oder Arylreste, wobei zwei gegebenenfalls vorliegende Reste auch miteinander — unter Einschluss des Boratoms — zu einem Ring geschlossen sein können. Die substituierenden Kohlenwasserstoffreste weisen insbesondere jeweils nicht mehr als 25 C-Atome auf. Bevorzugt enthält jeder dieser Reste nicht mehr als etwa 12 bis 15 C-Atome.

Eine besonders geeignete Klasse von Organoborverbindungen für die Herstellung der polymeren Initiatorkomponente sind Organobor-monohydridverbindungen, insbesondere Dialkylmonohydride. Typische Vertreter solcher Borverbindungen sind beispielsweise 9-Borabicyclo[3.3.1]nonan, Diisopinocampheylboran, Dicyclohexylboran, Thexylboran-(2,3-dimethyl-2-butylboran), 3,5-Dimethylborinan, Diisoamylboran. Unter diesen Verbindungen kann das zuerst genannte 9-Borabicyclo[3.3.1]nonan aus praktischen Gründen bevorzugt sein. Die vorstehend genannten Verbindungen können beispielsweise aus Natriumborhydrid und Bortrifluorid mit geeigneten Olefinen oder Diolefinen hergestellt werden. Auch können zur Darstellung Diboran, dessen Ether-, Amin- oder Sulfidkomplexe eingesetzt werden. Ganz allgemein gilt, dass bevorzugt mit solchen Organoborverbindungen zur Hydroborierung der Polymermatrix gearbeitet wird, die bei Raumtemperatur eine hinreichende thermische Stabilität aufweisen und gegenüber der Einwirkung von Luftsauerstoff möglichst stabil sind.

Eine Zusammenstellung der Herstellungsmöglichkeiten geeigneter Borverbindungen findet sich in der Monographie Herbert C. Brown, «Organic Synthesis via Boranes» (1975) Verlag John Wiley & Sons, New York.

Zur Hydroborierung werden die ungesättigten Oligomeren bzw. Polymeren unter vollkommenem Sauerstoffausschluss durch Reaktion mit den ausgewählten Borhydridverbindungen zweckmässig in Lösungsmitteln umgesetzt. Geeignet sind hier die bekannten Lösungsmittel für Organoborverbindungen, insbesondere Tetrahydrofuran, oder Polyether wie Diethylenglykoldimethylether aber auch Ester, Halogenkohlenwasserstoffe und dergleichen.

Die oligomeren bzw. polymeren Borinitiatoren gemäss der Erfindung können dann durch Abziehen des Lösungsmittels isoliert werden. Sie sind je nach der Monomerzusammensetzung und dem Molekulargewicht viskos bis fest. Ihre Lagerung erfolgt zweckmässigerweise im verschlossenen Gefäss, bevorzugt unter Inertgas beispielsweise Stickstoff. In Substanz sind diese polymeren bzw. oligomeren Boralkylhärter gegenüber Luft relativ stabil. Ausgewählte Starter können beispielsweise einen Tag lang an der Luft in einer offenen Schale gelagert werden, gleichwohl zeigen sie noch immer für die Aushärtung der olefinischen Komponente eine Reaktivität, die mit der frisch hergestellten oder unter Sauerstoffausschluss gelagerten Komponente praktisch identisch ist.

Zur Härtung der erfindungsgemässen Reaktionsmassen setzt man von den beschriebenen polymeren Borinitiatoren etwa 0,1 bis 40 Gew.-% insbesondere etwa 0,1 bis 30 Gew.-% — jeweils bezogen auf den zu polymerisierenden Anteil — ein. Bevorzugt werden die polymeren Härter in Mengen von etwa 0,5 bis 10 Gew.-% — bezogen auf den zu polymerisierenden Anteil — verwendet.

Als polymerisierbare Bestandteile können in den erfindungsgemässen Kunststoffmassen die zahlreichen bekannten Verbindungen mit polymerisierbarer ethylenischer Doppelbindung eingesetzt werden, die üblicherweise in beispielsweise Giessharzen, Füllstoffen und insbesondere in Reaktionsklebstoffen zur Verwendung kommen. Insbesondere geeignet sind dementsprechend die Ester von Acrylsäure und/oder $\alpha$-substituierten Acrylsäuren, wie Methacrylsäure — im folgenden als (Meth)-acrylsäureverbindungen bezeichnet — mit monovalenten oder polyvalenten insbesondere zweiwertigen Alkoholen. Geeignet sind aber auch andere bekannte Derivate der (Meth)-acrylsäure, insbesondere die entsprechenden Säureamide, die am Amidstickstoff auch zum Beispiel mit Kohlenwasserstoffresten substituiert sein können. Weitere mögliche Substituenten in $\alpha$-Stellung der (Meth)-acrylsäurederivate sind beispielsweise Halogen, insbesondere Chlor und/oder Brom, Cyan oder allgemein Alkylreste mit bis zu 10 C-Atomen.

Als Beispiele für (Meth)-acrylsäureester monovalenter Alkohole seien genannt: (Meth)-acrylsäuremethylester, (Meth)-acrylsäureethylester, (Meth)-acrylsäurebutylester, (Meth)-acrylsäureethylhexylester.

Beispiele entsprechender Ester mit polyvalenten Alkoholen sind solche mit Ethylenglykol, Diethylenglykol, Polyethylenglykol und Trimethylolpropan, Di- und Mono-(meth)-acrylsäureester von Glycerin, Di(meth)-acrylsäureester von Tri- und Tetraethylenglykol, von Di-, Tri-, Tetra- und Pentapropylenglykol, die Di-(meth)-acrylsäureester von ethoxyliertem oder propoxyliertem Diphenylolpropan. Auch kommen (Meth)-acrylsäureester von Alkoholen in Frage, die sich von Dihydroxymethyltricyclodecan ableiten oder auch solche, die auf Basis von Tricyclodecan hergestellt worden sind, wobei zwei alkoholische Funktionen im Ringsystem durch Umsetzung mit Dicarbonsäuren wie Maleinsäure oder auch Cyclohexandicarbonsäure oder Terephthalsäure verlängert sind.

Weiterhin sind verwendbar Umsetzungsprodukte des Diglycidylethers von Diphenylolpropan mit Methacrylsäure und/oder Acrylsäure. Auch sind Reaktionsprodukte von Diisocyanaten oder Triisocyanaten, zum Beispiel Toluylendiisocyanat,

Diphenylmethandiisocyanat, Isophorondiisocyanat, trimerisiertem Toluylenisocyanat und dergleichen mit Hydroxyalkyl-(meth)-acrylaten als polymerisierbare Bestandteile brauchbar.

Geeignet sind aber auch polymerisierbare Monomere wie Vinylacetat, Vinylhalogenide, zum Beispiel Vinylchlorid, Vinylbromid oder Vinylfluorid, Styrol, Diphenylbenzol, Crotonsäure- und Maleinsäureester oder die sogenannten, gegebenenfalls styrolisierten ungesättigten Polyesterharze. Diese zuletzt genannten Verbindungen werden im allgemeinen in Reaktionsklebstoffen nur in untergeordneter Menge, beispielsweise in Mengen bis zu 25 Gew.-% der polymerisierbaren Bestandteile, mitverwendet.

Ausserdem kommen in Betracht 2-Acryloyloxyethylphosphat, 2-Methacryloyloxyethylphosphat, Bis-2-acryloyloxyethylphosphat, Bis-2-methacryloyloxyethylphosphat, Tris-2-acryloyloxyethylphosphat, Tris-2-methacryloyloxyethylphosphat und Säureamide wie etwa Dimethylenbis(meth)-acrylamid, Tetramethylenbis(meth)acrylamid, Trimethylhexamethylenbis(meth)acrylamid, Tri-(meth)acryloyldiethylentriamin und dergleichen mehr.

Häufig enthalten die polymerisierbaren Massen zusätzlich zu den polymerisierbaren Komponenten vorgefertigte Polymerisate wie Polymethyl(meth)-acrylat, Copolymere von Methyl(meth)acrylat, Polychloropren, chlorsulfoniertes Polyethylen, Nitrilkautschuke und Urethane zur Verstärkung bzw. Elastifizierung und gleichzeitig als Verdikkungsmittel. Hierdurch wird die Verarbeitung der Massen, beispielsweise des Klebstoffes, erleichtert. Im einzelnen gelten die Angaben des einschlägigen Standes der Technik.

In vielen Fällen ist es zweckmässig oder notwendig, weitere Hilfsstoffe wie Füllstoffe, beispielsweise Quarzmehl oder dergleichen, zuzugeben. Schliesslich kann es zweckmässig sein, mit geeigneten Farbstoffen bzw. Pigmenten einzufärben.

Die erfindungsgemäss aerob härtenden Kunststoffmassen liegen in der Regel als Mehrkomponentensysteme vor, wobei eine Komponente durch den polymerisierbaren Anteil aus ethylenisch ungesättigten Monomeren, gegebenenfalls im Verschnitt mit polymeren Füllstoffen, Farbstoffen und dergleichen, gebildet ist, während eine getrennte zweite Komponente die polymeren Organoborverbindungen enthält. Diese Initiatorkomponente kann ausschliesslich aus der hydroborierten Polymermatrix bestehen, es ist aber auch möglich, diesen Starter im Verschnitt mit Verdünnungsmitteln einzusetzen. Als Verdünnungsmittel kommen insbesondere nichthydroborierte Polymermassen und/oder auch inerte Lösungs- bzw. Verdünnungsmittel in Betracht. Zur Stabilisierung der Härterkomponente gegenüber Luftsauerstoff wird die Abwesenheit oder die Gegenwart nur beschränkter Mengen an flüssigen Lösungsmitteln bevorzugt.

Die Erfindung betrifft damit insbesondere 2-Komponenten-Reaktionsklebstoffe, die neben einer an sich bekannten, die Klebstoffsubstanz bildenden Komponente als getrennt gelagerten Härter die oligomeren bzw. polymeren Organoborverbindungen der geschilderten Art aufweisen. Diese 2komponentigen Reaktionsmassen können in bekannter Weise verarbeitet werden, beispielsweise durch Vermischen von Härter und polymerisierbarer Reaktionsmasse vor dem Auftrag des Klebstoffs auf das zu verklebende Gut. Die erfindungsgemässen Massen sind aber auch für die Anwendung in den sogenannten «No-Mix-Klebstoffsystemen» geeignet.

Die neuen Klebstoffe zeichnen sich dadurch aus, dass sie bei Raumtemperatur eine hohe Härtungsgeschwindigkeit haben und bereits nach kurzer Zeit gute Festigkeiten auf einer Vielzahl von unterschiedlichen Oberflächen aufweisen. Hervorzuheben ist insbesondere, dass auch auf feuchten Oberflächen eine schnelle und gute Haftung erzielt wird. Die Klebstoffe können demnach eingesetzt werden als sogenannte Konstruktionsklebstoffe zur Verklebung von Metallen, Holz, Glas, Keramik, Kunststoffen. Darüber hinaus sind sie aber auch als dentalmedizinische Binde- und Füllmittel geeignet. Ausserdem eignen sie sich zum Verbinden bzw. Verkleben von hartem Gewebe, insbesondere von Knochen oder auch von Zähnen. Die erfindungsgemässen Klebstoffe können auch zum Verbinden von metallischen Oberflächen mit Knochen bzw. Zähnen oder vergleichbarem Hartgewebe eingesetzt werden.

Die in den folgenden Erörterungen verwendeten Warenzeichen werden als solche anerkannt.

### Beispiele

### A) Herstellung der oligomeren bzw. polymeren Olefine

#### Allgemeine Herstellvorschrift für Polymerisate:

Ein Edelstahlautoklav mit Rührer und Temperaturmesseinheit wird mit Acrylsäureester, Lösungsmittel (Tetrahydrofuran — THF), Radikalstarter (Azoisobuttersäurenitril — AIBN) und Regler (Thiophenol) beschickt und verschlossen. Der Autoklav wird 3mal mit Stickstoff 4,9 bar (5 atm) gespült und anschliessend über eine Dosierbombe mit Butadien(-1,3) beschickt. Die Polymerisation wird 7 Stunden bei $60 \pm 1°C$ unter Rühren durchgeführt. Der Maximaldruck betrug 8,9 bar (9 atm). Der Ansatz wird dem Autoklaven entnommen und Lösungsmittel sowie nicht reagiertes Monomer am Rotationsverdampfer entfernt. Die Zusammensetzungen der Ansätze und Polymereigenschaften sind der Tabelle A (A1-A4) zu entnehmen.

#### Allgemeine Herstellvorschrift für Polykondensate:

#### Polyester

In einem Dreihalskolben mit Rührer und Destillationsbrücke werden Alkenylbernsteinsäureanhydrid (ABSA) und Diol vorgelegt. Unter Stickstoff wird schnell auf 150°C und dann im Verlauf von 3 Stunden von 150°C auf 200°C hochgeheizt. Dabei spaltet sich bereits der grösste Anteil des Reaktionswassers, der den Umsatz der Esterkondensation anzeigt, ab. Man lässt den Ansatz auf etwa 150°C abkühlen, evakuiert vorsichtig auf 13 mbar (10 Torr) und vervollständigt den Umsatz bei 200°C und 13 mbar (10 Torr). Das Produkt wird heiss abgefüllt. Die Zusammensetzung der Ansätze und die Polymereigenschaften sind der Tabelle A (A6-A10) zu entnehmen.

*Polyamide*

In einen Dreihalskolben mit Rührer und Destillationsaufsatz wird das Amin vorgelegt und das Alkenylbernsteinsäureanhydrid (ABSA) zügig unter Wasserkühlung zugegeben, wobei darauf geachtet werden muss, dass die Reaktionstemperatur um 100°C liegt, damit das Ammoniumsalz flüssig bleibt. Das Ammoniumsalz wird unter Stickstoff im Verlauf von 2 Stunden langsam auf 200°C hochgeheizt. Dabei spaltet sich bereits der grösste Anteil des Reaktionswassers, der den Umsatz der Amidbildung anzeigt, ab. Man lässt den Ansatz auf etwa 150°C abkühlen, evakuiert vorsichtig auf 10 Torr und vervollständigt die Amidbildung bei 200°C und 13 mbar (10 Torr). Das Produkt wird heiss abgefüllt. Die Zusammensetzung der Ansätze und die Oligomer- beziehungsweise Polymereigenschaften sind der Tabelle A (A11-A15) zu entnehmen.

*(Tabelle A auf der nächsten Seite)*

*B) Herstellung der oligomeren bzw. polymeren Boralkylstarter*

Zur Befreiung von Restsauerstoff werden die Oligo- bzw. Polyolefine in der gleichen Menge THF gelöst und das Lösungsmittel im Vakuum bei $1{,}3 \cdot 10^{-4}$ mbar ($10^{-4}$ Torr) abgezogen. In einer Glovebox werden erneut gleiche Gewichtsteile frisch destilliertes, entgastes THF zugegeben und die oligomeren bzw. polymeren Olefine gelöst. Unter vollständigem Sauerstoffausschluss werden die in der Tabelle B aufgelisteten Mengen an 9-Borabicyclo[3.3.1]-nonan (9-BBN) zugegeben und das Gemisch so lange gerührt, bis das 9-BBN quantitativ in Lösung gegangen ist. Im Anschluss erhitzt man 1 h unter Rühren auf 60°C. Das THF wird im Vakuum abgezogen und das Vorratsgefäss verschlossen. Die Entnahme von Proben erfolgt unter Schutzgass und völligem Sauerstoffausschluss.

*Tabelle B*

*Übersicht über die dargestellten oligomeren und polymeren Boralkyle*

| Nr. | Eingesetztes Olefin (g) | 9-BBN | Modifizierungs-grad an der Doppelbindung (%) | Eingenschaften |
|---|---|---|---|---|
| B 1 | A 1 / 10 | 1,0 | 22 | hochviskos |
| B 2 | A 2 / 10 | 1,0 | 18 | viskos |
| B 3 | A 3 / 10 | 0,72 | 100 | hochviskos |
| B 4 | A 4 / 10 | 0,4 | 100 | zähviskos |
| B 5 | Poly(cis-1,4-butadien-1,3)[1]/10 | 0,7 | 3 | viskos |
| B 6 | A 6 / 50 | 19,5 | 100 | homogen, zäh, orange |
| B 7 | A 7 / 50 | 17 | 100 | homogen, hochviskos, hellgelb |
| B 8 | A 8 / 50 | 14,6 | 100 | homogen, hochviskos, hellgelb |
| B 9 | A 9 / 50 | 15,4 | 50 | homogen, hochviskos, rotbraun |
| B 10 | A 10 / 50 | 4,8 | 100 | Pulver, hellgelb |
| B 11 | A 11 / 50 | 17,7 | 100 | homogen, fest, orange |
| B 12 | A 12 / 50 | 15,8 | 100 | homogen, fest, hellgelb |
| B 13 | A 13 / 50 | 21,4 | 100 | homogen, fest, orange |
| B 14 | A 14 / 50 | 13,5 | 100 | homogen, zäh, gelb |
| B 15 | A 15 / 50 | 17,7 | 100 | homogen, viskos, gelb-orange |

[1] Polyol 130 der CWH, Hüls.

Tabelle A

Übersicht über die dargestellten oligomeren und polymeren Olefine/Polymerisate

| Nr. | Polymerisationsansatz | | | | | Ergebnisse | | | |
|---|---|---|---|---|---|---|---|---|---|
| | Eingesetzte Mengen Monomer (g) | | AIBN (g) | Thiophenol (g) | THF (g) | Mol-% Butadien | | Eigenschaften des Polymer | Umsatz (%) |
| | | | | | | im Monomergemisch | im Polymer[1] | | |
| A 1 | Butadien 21,6 | Acrylsäuremethylester 137,8 | 1,6 | 0,92 | 35 | 20 | 38 | hochviskoses Öl | 20 |
| A 2 | Butadien 108 | Acrylsäuremethylester 689 | 8,0 | 11,60 | 1400 | 20 | 44 | viskoses Öl | 17 |
| A 3 | Butadien 270 | Acrylsäurebutylester 641 | 8,0 | 11,60 | 1700 | 50 | 7,2 | hochviskoses Öl | 73 |
| A 4 | Butadien | Acrylsäurebutylester 513 | 4,0 | — | 1700 | 20 | 4,2 | sehr hochviskoses Öl | 85 |

[1] Bestimmung erfolgte durch $^1$H-NMR

Tabelle A (Fortsetzung)

| Nr. | Polymerisationsansatz | | Ergebnisse | | | |
|---|---|---|---|---|---|---|
| | Eingesetzte Menge Edukt (g) | | Eigenschaften des Polymer | Säurezahl | Jodzahl | Mole Doppelbindung (100 g) Oligomer/Polymer |
| A 6 | Iso $C_8$ ABSA 63,3 | Hexamethylenglycol 35,4 | viskos, bräunlich | 48 | 77,7 | 0,32 |
| A 7 | $C_{10}$ ABSA 239 | Hexamethylenglycol 118 | viskos, hellgelb | 27 | 72,9 | 0,29 |
| A 8 | $C_{16}$ ABSA 323 | Hexamethylenglycol 118 | leicht viskos, bräunlich | 43 | 55,0 | 0,24 |
| A 9 | Maleinsäure 232,16 | Hexamethylenglycol 236 | fest, weiss | 0 | 128 | 0,5 |
| A 10 | Maleinsäure 58,05 Adipinsäure 292,3 | Hexamethylenglycol 292 | fest, weiss | 4,2 | 20 | 0,08 |

_Tabelle A (Fortsetzung)_

| Nr. | Polymerisationsansatz Eingesetzte Menge Edukt (g) | Ergebnisse Eigenschaften des Polymer | Aminzahl | Jodzahl | Mole Doppelbindung (100 g) Oligomer/Polymer |
|---|---|---|---|---|---|
| A 11 | Iso $C_8$ ABSA 58,5 / Trimethylhexamethylendiamin 43,8 | viskos, braun | 115 | 69,3 | 0,29 |
| A 12 | $C_{10}$ ABSA 239 / Trimethylhexamethylendiamin 158 | niedrigviskos, hellbraun | 115 | 76,2 | 0,26 |
| A 13 | $C_{10}$ ABSA 169,7 / Trimethylhexamethylendiamin 75,4 | viskos, hellbraun | 29 | 78,3 | 0,35 |
| A 14 | $C_{16}$ ABSA 286 / Trimethylhexamethylendiamin 139,9 | niedrigviskos, braun-grün | 98 | 60,7 | 0,22 |
| A 15 | $C_{16}$ ABSA 195 / Trimethylhexamethylendiamin 67,2 | viskos, braun-grün | 31 | 58,3 | 0,29 |

C) _Verwendung der oligomeren bzw. polymeren Boralkylen als Härter für Monomerklebstoffe_

_Allgemeine Vorschrift_

In einem Becherglas wurden 40 g Polymethacrylsäuremethylester (PMMA, handelsübliches Pulver «Plexigum MB 319» der Firma Röhm, Darmstadt) in 45 g Methacrylsäuremethylester (MMA) und 5 g Methacrylsäure (MAS) unter Rühren gelöst. Zu jeweils 5 g dieser Mischung wurden unter weiterem intensiverem Rühren zwischen 1,5 und 23 Gew.-% der bereits unter B beschriebenen oligomeren bzw. polymeren Boralkylstarter (siehe Tabellen C1-C15) zugegeben. Die Topfzeiten der Mischungen variieren zwischen 1 und 13 Minuten. Mit diesen Klebstoffen wurden innerhalb der Topfzeit sandgestrahlte und entfettete Eisenbleche verklebt und nach 24 h die Festigkeiten im Zugscherversuch nach DIN 53281/3 gemessen. Die Ergebnisse sind in den Tabellen 1-5 zusammengestellt.

Zum Nachweis der hohen Stabilität der dargestellten oligomeren bzw. polymeren Boralkyle gegenüber Luftsauerstoff wurden sie in einer weiteren Versuchsreihe im offenen Gefäss zwischen 24 und 72 h an der Luft gelagert und im Anschluss als Härter eingesetzt und getestet. Die Topfzeiten und Zugscherfestigkeiten sind in den Tabellen C 1-C 15 in Klammern gesetzt.

_Tabelle C 1_

_Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Borarkyl aus Beispiel B 1_

| Härterkonzentration (Gew.-%) | Topfzeit (min) | Zugscherfestigkeiten ($Nmm^{-2}$) |
|---|---|---|
| 1,5 | 10 | 19 |
| 3 | 10 | 20 |
| 5 | 5 | 24 |
| 10 | 5 | 12 |
| 23 | 2 | 9 |

_Tabelle C 2_

_Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 2_

| Härterkonzentration (Gew.-%) | Topfzeit (min) | Zugscherfestigkeiten ($Nmm^{-2}$) |
|---|---|---|
| 1,5 | 10 (12) | 21 (10) |
| 3 | 5 ( 7) | 25 (24) |
| 5 | 5 ( 5) | 29 (30) |
| 10 | 5 ( 5) | 27 (28) |
| 23 | 5 ( 4) | 13 (24) |

Bei den in Klammern gesetzten Messergebnissen wurde der Boralkylhärter vor der Verwendung 24 h bei Raumtemperatur an der Luft gelagert.

*Tabelle C 3*

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 3*

| Härterkonzen-tration (Gew.-%) | Topfzeit (min) | Zugscherfestig-keiten (Nmm$^{-2}$) |
|---|---|---|
| 1,5 | 8 (11) | 16 ( 5) |
| 3 | 9 ( 8) | 27 (14) |
| 5 | 4 ( 5) | 29 (28) |
| 10 | 3 ( 4) | 28 (27) |
| 23 | 3 ( 3) | 26 (24) |

Bei den in Klammern gesetzten Messergebnissen wurde der Boralkylhärter vor der Verwendung 72 h bei Raumtemperatur an der Luft gelagert.

*Tabelle C 4*

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem polymeren Boralkyl aus Beispiel B 4*

| Härterkonzen-tration (Gew.-%) | Topfzeit (min) | Zugscherfestig-keiten (Nmm$^{-2}$) |
|---|---|---|
| 1,5 | 4 (11) | 0 ( 0 ) |
| 3 | 2,5 (11) | 16 ( 0,4) |
| 5 | 2 ( 9) | 15 ( 0,8) |
| 10 | 1,5 ( 3) | 19 ( 3,5) |
| 23 | 1,5 ( 2) | 9 (13 ) |

Bei den in Klammern gesetzten Messergebnissen wurde der Boralkylhärter vor der Verwendung 24 h bei Raumtemperatur an der Luft gelagert.

*Tabelle C 5*

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 5*

| Härterkonzen-tration (Gew.-%) | Topfzeit (min) | Zugscherfestig-keiten (Nmm$^{-2}$) |
|---|---|---|
| 1,5 | 2,5 (8) | 9 ( 1) |
| 3 | 3 (7) | 18 ( 8) |
| 5 | 2 (2) | 21 (16) |
| 10 | 2 (2) | 17 (17) |
| 23 | 1 (1) | 2 ( 3) |

Bei den in Klammern gesetzten Messergebnissen wurde der Boralkylhärter vor der Verwendung 24 h bei Raumtemperatur an der Luft gelagert.

*Tabelle C 6*

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 6*

| Härterkonzen-tration (Gew.-%) | Topfzeit (min) | Zugscherfestig-keiten (Nmm$^{-2}$) |
|---|---|---|
| 1,5 | 7 | 17 |
| 3 | 5 (6 ) | 26 (26) |
| 5 | 4 (4,5) | 27 (28) |
| 10 | 3 (4 ) | 26 (30) |
| 23 | 2,5 (3 ) | 20 (24) |

Bei den in Klammern gesetzten Messergebnissen wurde der Boralkylhärter vor der Verwendung 48 h bei Raumtemperatur an der Luft gelagert.

*Tabelle C 7*

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 7*

| Härterkonzen-tration (Gew.-%) | Topfzeit (min) | Zugscherfestig-keiten (Nmm$^{-2}$) |
|---|---|---|
| 1,5 | 6 | 9 |
| 3 | 5 (6 ) | 23 (21) |
| 5 | 4 (5 ) | 25 (27) |
| 10 | 4 (4 ) | 22 (23) |
| 23 | 2,5 (0,5) | 15 (18) |

Bei den in Klammern gesetzten Messergebnissen wurde der Boralkylhärter vor der Verwendung 24 h bei Raumtemperatur an der Luft gelagert.

*Tabelle C 8*

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 8*

| Härterkonzen-tration (Gew.-%) | Topfzeit (min) | Zugscherfestig-keiten (Nmm$^{-2}$) |
|---|---|---|
| 1,5 | 8 | 12 |
| 3 | 6 (8 ) | 20 (16) |
| 5 | 4 (5,5) | 24 (26) |
| 10 | 3,5 (4 ) | 21 (24) |
| 23 | 2 (2 ) | 13 (16) |

Bei den in Klammern gesetzten Messergebnissen wurde der Boralkylhärter vor der Verwendung 48 h bei Raumtemperatur an der Luft gelagert.

### Tabelle C 9

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 9*

| Härterkonzentration (Gew.-%) | Topfzeit (min) | Zugscherfestig- keiten (Nmm$^{-2}$) |
|---|---|---|
| 1,5 | 12 (9 ) | 25 (23) |
| 3 | 12 (5,5) | 30 (27) |
| 5 | 11,5 (4,5) | 32 (29) |
| 10 | 8,5 (3,5) | 26 (29) |
| 23 | 5 (3 ) | 20 (27) |

Bei den in Klammern gesetzten Messergebnissen wurde der Boralkylhärter vor der Verwendung 24 h bei Raumtemperatur an der Luft gelagert.

### Tabelle C 10

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 10*

| Härterkonzentration (Gew.-%) | Topfzeit (min) | Zugscherfestig- keiten (Nmm$^{-2}$) |
|---|---|---|
| 1,5 | 7 ( 9,5) | 27 ( 3) |
| 3 | 6,5 (13,5) | 29 (11) |
| 5 | 6,5 (12 ) | 28 (24) |
| 10 | 6,5 (11 ) | 29 (21) |
| 23 | 6 (20 ) | 27 ( 1) |

Bei den in Klammern gesetzten Messergebnissen wurde der Boralkylhärter vor der Verwendung 24 h bei Raumtemperatur an der Luft gelagert.

### Tabelle C 11

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 11*

| Härterkonzentration (Gew.-%) | Topfzeit (min) | Zugscherfestig- keiten (Nmm$^{-2}$) |
|---|---|---|
| 1,5 | 4 (6) | 17 (17) |
| 3 | 4 (5) | 18 (24) |
| 5 | 3 (5) | 16 (27) |
| 10 | 2 (3) | 8 (21) |
| 23 | 1,5 (2) | 4 (13) |

Bei den in Klammern gesetzten Messergebnissen wurde der Boralkylhärter vor der Verwendung 24 h bei Raumtemperatur an der Luft gelagert.

### Tabelle C 12

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 12*

| Härterkonzentration (Gew.-%) | Topfzeit (min) | Zugscherfestig- keiten (Nmm$^{-2}$) |
|---|---|---|
| 1,5 | 12 (11) | 14 (13) |
| 3 | 9 (10) | 27 (17) |
| 5 | 7 ( 5) | 30 (26) |
| 10 | 5 ( 4) | 26 (23) |
| 23 | 3 ( 3) | 20 (11) |

Bei den in Klammern gesetzten Messergebnissen wurde der Boralkylhärter vor der Verwendung 24 h bei Raumtemperatur an der Luft gelagert.

### Tabelle C 13

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 13*

| Härterkonzentration (Gew.-%) | Topfzeit (min) | Zugscherfestig- keiten (Nmm$^{-2}$) |
|---|---|---|
| 1,5 | 8 (8 ) | 20 (20) |
| 3 | 6 (6 ) | 24 (25) |
| 5 | 5 (5 ) | 25 (28) |
| 10 | 4 (4 ) | 18 (20) |
| 23 | 2,5 (2,5) | 1 (12) |

Bei den in Klammern gesetzten Messergebnissen wurde der Boralkylhärter vor der Verwendung 24 h bei Raumtemperatur an der Luft gelagert.

### Tabelle C 14

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 14*

| Härterkonzentration (Gew.-%) | Topfzeit (min) | Zugscherfestig- keiten (Nmm$^{-2}$) |
|---|---|---|
| 1,5 | 8 (12 ) | 8 (20) |
| 3 | 6 (10 ) | 15 (18) |
| 5 | 5 (10 ) | 18 (18) |
| 10 | 3,5 ( 4 ) | 17 (16) |
| 23 | 2 ( 3,5) | 11 (12) |

Bei den in Klammern gesetzten Messergebnissen wurde der Boralkylhärter vor der Verwendung 24 h bei Raumtemperatur an der Luft gelagert.

*Tabelle C 15*

*Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit dem oligomeren Boralkyl aus Beispiel B 15*

| Härterkonzentration (Gew.-%) | Topfzeit (min) | Zugscherfestigkeiten (Nmm$^{-2}$) |
|---|---|---|
| 1,5 | 8 (8) | 21 (18) |
| 3 | 5,5 (6) | 24 (28) |
| 5 | 3,5 (4) | 19 (23) |
| 10 | 4,5 (4) | 15 (20) |
| 23 | 2,5 (3) | 3,6 (16) |

Bei den in Klammern gesetzten Messergebnissen wurde der Boralkylhärter vor der Verwendung 24 h bei Raumtemperatur an der Luft gelagert.

In völliger Analogie wurden Aluminium- und Buchenholzprüfkörper einfach überlappend verklebt und die Zugscherfestigkeiten ermittelt.

Zusätzlich wurden Klebstoffe auf der Basis Triethylenglykoldimethacrylat und Bisphenol A-dimethacrylat (Diacryl 101, Firma AKZO Chem.) mit den Härtern aus den Beispielen B2 und B3 polymerisiert. Mit ihnen wurden Eisen- und Aluminiumbleche einfach überlappend verklebt. Die Ergebnisse der Zugscherfestigkeitsmessungen sind der Tabelle C16 zu entnehmen. Die Prüfkörper wurden zwischen Verkleben und Zerreissen 24 h bei Raumtemperatur gelagert.

*Tabelle C 16*

*Zugscherfestigkeiten bei der Verklebung von Methacrylatklebstoffen mit den oligomeren Boralkylen aus Beispielen 2 und B 3*

| Klebstoff | | | Härter | | Verklebtes Material | Zugscherfestigkeit (Nmm$^{-2}$) |
|---|---|---|---|---|---|---|
| Monomer 1 | Monomer 2 | Polymer | Nr. | Konz./ Gew.-% | | |
| Methylmethacrylat 45 g | Methacrylsäure 5 g | Polymethylmethacrylat 40 g | B 2 | 5 | Aluminium | 8 |
| | | | | | Buchenholz | 3 |
| | | | B 3 | 5 | Aluminium | 14 |
| Triethylenglycoldimethacrylat 75 g | Methacrylsäure 5 g | Polymethylmethacrylat 20 g | B 2 | 3 | Eisen | 14 |
| | | | | | Aluminium | 9 |
| | | | B 3 | 3 | Eisen | 14 |
| Diacryl 101 75 g | Methacrylsäure 5 g | Polymethylmethacrylat 20 g | B 2 | 3 | Eisen | 10 |
| | | | | | Aluminium | 11 |
| | | | B 3 | 3 | Eisen | 10 |
| | | | | | Aluminium | 10 |

**Patentansprüche**

1. Aerobhärtende Kunststoffmassen, wie Giessharze, Füllstoffe und insbesondere Reaktionsklebstoffe auf Basis polymerisierbare ethylenische Doppelbindungen enthaltender Systeme und Organo-Borverbindungen als Polymerisationsinitiatoren, dadurch gekennzeichnet, dass sie polymere Organo-Borverbindungen enthalten, die als Substituenten an einer gegen Luftzutritt stabilen Polymermatrix Organo-Borreste aufweisen, wobei als Polymermatrix ein ethylenische Doppelbindungen aufweisendes Oligomeres bzw. Polymeres vorliegt, dessen Doppelbindungen wenigstens anteilsweise durch Einführung der borhaltigen Substituenten mittels Hydroborierung zu gesättigten Bindungen umgewandelt sind.

2. Kunststoffmassen nach Anspruch 1 dadurch gekennzeichnet, dass in der polymeren Organo-Borverbindung die borhaltigen Reste über vorwiegend B−C-Bindungen an die Polymermatrix gebunden sind.

3. Kunststoffmassen nach Ansprüchen 1 und 2 dadurch gekennzeichnet, dass in der polymeren Organo-Borverbindung als Substituenten Organo-Borreste mit Alkyl-, Cycloalkyl- und/oder Arylgruppen vorliegen, die auch noch eine Borhydridbindung aufweisen können.

4. Kunststoffmassen nach Ansprüchen 1 bis 3 dadurch gekennzeichnet, dass die einzelnen Kohlenwasserstoffreste in den die Polymermatrix substituierenden Organo-Borresten jeweils bis zu 25 C-Atome, vorzugsweise bis zu 15 C-Atome aufweisen.

5. Kunststoffmassen nach Ansprüchen 1 bis 4 dadurch gekennzeichnet, dass die Polymermatrix ein mittleres Molekulargewicht im Bereich von etwa 150 bis 3 000 000, vorzugsweise im Bereich von etwa 300 bis 500 000 und insbesondere im Bereich von etwa 300 bis 10 000 aufweist.

6. Kunststoffmassen nach Ansprüchen 1 bis 5 dadurch gekennzeichnet, dass wenigstens 30%, vorzugsweise wenigstens 50% und insbesondere wenigstens 80% der in der Polymermatrix ursprünglich vorhandenen ethylenischen Doppelbindungen hydroboriert sind.

7. Kunststoffmassen nach Ansprüchen 1 bis 6 dadurch gekennzeichnet, dass die Polymermatrix überwiegend C—C-Bindungen in den Polymerketten aufweist.

8. Kunststoffmassen nach Ansprüchen 1 bis 7 dadurch gekennzeichnet, dass die Polymermatrix geradkettige oder verzweigtkettige Struktur aufweist.

9. Kunststoffmassen nach Ansprüchen 1 bis 8 dadurch gekennzeichnet, dass die Polymermatrix ein ethylenische Doppelbindungen aufweisendes Polymerisat oder Copolymerisat, Polykondensat oder ein entsprechendes Polyadditionsprodukt ist.

10. Kunststoffmassen nach Ansprüchen 1 bis 9 dadurch gekennzeichnet, dass die Polymermatrix vor der Hydroborierung eine Jodzahl im Bereich von etwa 1 bis 500, vorzugsweise im Bereich von etwa 5 bis 100 und insbesondere im Bereich von etwa 8 bis 50 aufweist.

11. Kunststoffmassen nach Ansprüchen 1 bis 10 dadurch gekennzeichnet, dass die der Hydroborierung unterworfenen Doppelbindungen der polymeren Organo-Borverbindung in der Hauptkette und/oder in Seitenketten vorliegen.

12. Kunststoffmassen nach Ansprüchen 1 bis 11 dadurch gekennzeichnet, dass als Polymermatrix Oligomere oder Polymere mit olefinisch ungesättigten Doppelbindungen, hergestellt durch Dien-Polymerisation oder Copolymerisation von Dienen mit olefinisch ungesättigten Monomeren, vorliegen.

13. Kunststoffmassen nach Ansprüchen 1 bis 11 dadurch gekennzeichnet, dass als Polymermatrix olefinisch ungesättigte Polyester eingesetzt worden sind, deren Doppelbindungen bevorzugt wenigstens anteilsweise seitenständig vorliegen.

14. Kunststoffmassen nach Ansprüchen 1 bis 13 dadurch gekennzeichnet, dass sie als 2-Komponenten-Klebstoffe vorliegen, die neben einer polymerisierbare Monomere mit ethylenischen Doppelbindungen enthaltenden Komponente (A) das Startsystem (B) auf Basis der polymeren Organo-Borverbindung enthalten.

15. Kunststoffmassen nach Ansprüchen 1 bis 14 dadurch gekennzeichnet, dass die Komponenten A und/oder B bei Raumtemperatur fliess- bzw. streichfähig sind.

16. Kunststoffmassen nach Ansprüchen 14 und 15 dadurch gekennzeichnet, dass die Komponente A ein radikalisch polymerisierbares Klebersystem auf Basis von Derivaten — vorzugsweise Estern und/oder Säureamiden — der Acrylsäure und/oder von α-substituierten Acrylsäuren ist, das vorzugsweise diese polymerisierbaren Komponenten in homogener Abmischung mit Polymeren enthält.

17. Verwendung der Kunststoffmassen nach Ansprüchen 1 bis 16 als Reaktionsklebstoffe zum Verbinden von Metall, Holz, Glas, Keramik und/oder Kunststoffen, als chirurgisches Bindemittel zum Verbinden von hartem Gewebe, insbesondere Knochen, gewünschtenfalls mit Metallen oder Kunststoffen, oder als dentalmedizinisches Binde- und Füllmaterial.

**Claims**

1. Aerobically curing plastics compositions, such as cast resins, fillers and more particularly reactive adhesives based on systems containing polymerizable ethylenic double bonds and organoboron compounds as polymerization initiators, characterized in that they contain polymeric organoboron compounds containing organoboron residues as substituents on a polymer matrix stable against air, the polymer matrix being an oligomer or polymer containing ethylenic double bonds which are at least partly converted into saturated bonds by introduction of the boron-containing substituents by hydroboration.

2. Plastics compositions as claimed in Claim 1, characterized in that, in the polymeric organoboron compound, the boron-containing residues are attached to the polymer matrix by predominantly B—C-bonds.

3. Plastics compositions as claimed in Claims 1 and 2, characterized in that organoboron residues containing alkyl, cycloalkyl and/or aryl groups and, optionally, a borohydride bond are present as substituents in the polymeric organoboron compound.

4. Plastics compositions as claimed in Claims 1 to 3, characterized in that the individual hydrocarbon radicals in the organoboron residues substituting the polymer matrix each contain up to 25 C-atoms and preferably up to 15 C-atoms.

5. Plastics compositions as claimed in Claims 1 to 4, characterized in that the polymer matrix has an average molecular weight in the range from about 150 to 3,000,000, preferably in the range from about 300 to 500,000 and more preferably in the range from about 300 to 10,000.

6. Plastics compositions as claimed in Claims 1 to 5, characterized in that at least 30%, preferably at least 50% and more preferably at least 80% of the ethylenic double bonds originally present in the polymer matrix are hydroborated.

7. Plastics compositions as claimed in Claims 1 to 6, characterized in that the polymer matrix contains predominantly C—C-bonds in the polymer chains.

8. Plastics compositions as claimed in Claims 1 to 7, characterized in that the polymer matrix has a straight-chain or branched-chain structure.

9. Plastics compositions as claimed in Claims 1 to 8, characterized in that the polymer matrix is a polymer or copolymer or polycondensate containing ethylenic double bonds or a corresponding polyadduct.

10. Plastics compositions as claimed in Claims 1 to 9, characterized in that, before hydroboration, the polymer matrix has an iodine number of from about 1 to 500, preferably from about 5 to 100 and more preferably from about 8 to 50.

11. Plastics compositions as claimed in Claims 1 to 10, characterized in that the double bonds of the polymeric organoboron compound which are subjected to hydroboration are present in the main chain and/or in side chains.

12. Plastics compositions as claimed in Claims 1 to 11, characterized in that oligomers or polymers containing olefinically unsaturated double bonds, produced by diene polymerization or by copolymerization of dienes with olefinically unsaturated monomers, are present as the polymer matrix.

13. Plastics compositions as claimed in Claims 1 to 11, characterized in that olefinically unsaturated polyesters of which the double bonds are preferably at least partly situated in side chains are used as the polymer matrix.

14. Plastics compositions as claimed in Claims 1 to 13, characterized in that they are present as 2-component adhesives which, in addition to a component (A) containing polymerizable monomers having ethylenic double bonds, contain the starting system (B) based on the polymeric organoboron compound.

15. Plastics compositions as claimed in Claims 1 to 14, characterized in that the components A and/or B are free-flowing or spreadable at room temperature.

16. Plastics compositions as claimed in Claims 14 and 15, characterized in that component A is a radically polymerizable adhesive system based on derivatives, preferably esters and/or acid amides, of acrylic acid and/or α-substituted acrylic acids which preferably contains these polymerizable components in homogenous admixture with polymers.

17. The use of the plastics compositions claimed in Claims 1 to 16 as reactive adhesives for bonding metals, wood, glass, ceramics and/or plastics, as a surgical binder for bonding hard tissue, especially bones, if desired to metals or plastics or as a dental binder and filler.


**Revendications**

1. Masses de matières synthétiques à durcissement aérobie, telles que des résines de coulée, des matières de charge et en particulier des adhésifs réactifs à base de systèmes polymérisables contenant des doubles liaisons éthyléniques et de composés organiques de bore en tant qu'initiateurs de polymérisation, caractérisées en ce qu'elles contiennent des composés organiques de bore polymères qui présentent comme substituants, sur une matrice polymère stable au contact de l'air, des radicaux organiques de bore, en ayant comme matrice polymère un oligomère ou polymère présentant des doubles liaisons éthyléniques, dont ses doubles liaisons, tout au moins en partie, sont converties par l'introduction des substituants renfermant du bore en des liaisons saturées en recourant à une hydroboruration.

2. Masses de matières synthétiques selon la revendication 1, caractérisées en ce que dans le composé organique de bore polymère, les radicaux contenant du bore sont reliés à la matrice polymère de manière prépondérante par des liaisons $B-C$.

3. Masses de matières synthétiques selon les revendications 1 et 2, caractérisées en ce que dans le composé organique de bore polymère, il y a comme substituants des radicaux organiques de bore avec groupes alcoyle, cycloalcoyle et/ou aryle, qui peuvent aussi présenter encore une liaison borohydrure.

4. Masses de matières synthétiques selon les revendications 1 à 3, caractérisées en ce que les divers radicaux hydrocarbonés dans les radicaux organiques du bore qui substituent la matrice polymère présentent chacun jusqu'à 25 atomes de carbone, de préférence jusqu'à 15 atomes de carbone.

5. Masses de matières synthétiques selon les revendications 1 à 4, caractérisées en ce que la matrice polymère présente un poids moléculaire moyen dans l'intervalle d'environ 150 à 3 000 000, de préférence dans l'intervalle d'environ 300 à 500 000 et en particulier dans l'intervalle d'environ 300 à 10 000.

6. Masses de matières synthétiques selon les revendications 1 à 5, caractérisées en ce qu'au moins 30%, de préférence au moins 50% et en particulier au moins 80% des doubles liaisons éthyléniques présentes initialement dans la matrice polymère sont hydroborurées.

7. Masses de matières synthétiques selon les revendications 1 à 6, caractérisées en ce que la matrice polymère présente en prépondérance des liaisons $C-C$ dans les chaînes polymères.

8. Masses de matières synthétiques selon les revendications 1 à 7, caractérisées en ce que la matrice polymère présente une structure à chaîne droite ou ramifiée.

9. Masses de matières synthétiques selon les revendications 1 à 8, caractérisées en ce que la matrice polymère est un polymère ou copolymère, un polycondensat ou un produit de polyaddition approprié, qui présentent des doubles liaisons éthyléniques.

10. Masses de matières synthétiques selon les revendications 1 à 9, caractérisées en ce que la matrice polymère, avant l'hydroboruration, présente un indice d'iode dans l'intervalle d'environ 1 à 500, de préférence dans l'intervalle d'environ 5 à 100 et en particulier dans l'intervalle d'environ 8 à 50.

11. Masses de matières synthétiques selon les revendications 1 à 10, caractérisées en ce que les doubles liaisons du composé organique de bore polymère qui sont soumises à l'hydroboruration se trouvent dans la chaîne principale et/ou dans des chaînes latérales.

12. Masses de matières synthétiques selon les revendications 1 à 11, caractérisées en ce que comme matrice polymère il y a des oligomères ou des polymères avec doubles liaisons oléfiniquement insaturées préparés par polymérisation de diènes ou copolymérisation de diènes avec des monomères oléfiniquement insaturés.

13. Masses de matières synthétiques selon les revendications 1 à 11, caractérisées en ce que comme matrice polymère on utilise des polyesters oléfiniquement insaturés dont les doubles liaisons, tout au moins en partie, se présentent en situation latérale.

14. Masses de matières synthétiques selon les revendications 1 à 13, caractérisées en ce qu'elles se présentent sous forme d'adhésifs à 2 composants qui, à côté d'un composant (A) contenant des monomères polymérisables avec doubles liaisons éthyléniques, contiennent le système initiateur (B) à base du composé organique de bore polymère.

15. Masses de matières synthétiques selon les revendications 1 à 14, caractérisées en ce que les composants A et/ou B sont fluides ou brossables à la température ordinaire.

16. Masses de matières synthétiques selon les revendications 14 et 15, caractérisées en ce que le composant A est un système adhésif polymérisable par la voie radicalaire, à base de dérivés — de préférence des esters et/ou amides d'acides — de l'acide acrylique et/ou d'acides acryliques α-substitués, qui de préférence contient ces composants polymérisables en mélange homogène avec des polymères.

17. Utilisation des masses de matières synthétiques selon les revendications 1 à 16 comme adhésifs réactifs pour relier du métal, du bois, du verre, de la céramique et/ou des matières synthétiques, comme liant chirurgical pour relier du tissu dur, en particulier des os, éventuellement avec des métaux ou des matières synthétiques, ou comme matière liante et de charge en médecine dentaire.